# EUROPEAN PATENT SPECIFICATION

(11) **EP 2 344 391 B1**
(45) Date of publication and mention of the grant of the patent: **27.05.2015**
(21) Application number: 09819972.2
(22) Date of filing: 09.10.2009
(51) Int. Cl.: A61L 2/26, B65B 61/14, A61B 19/02, B65D 75/56, B65D 71/00

(54) **MODULAR COVER FOR STERILISATION WRAPPER**
MODULARE ABDECKUNG FÜR STERILISIERBEHÄLTER
COUVERCLE MODULAIRE POUR RECIPIENT DE STERILISATION

(30) Priority: 10.10.2008 US 104302 P
(43) Date of publication of application: 20.07.2011
(73) Proprietor: Depuy (Ireland), Ringaskiddy Cork (IE)
(72) Inventor: THOMAS, Kyle, Denver, CO 80211 (US); REEVE, Michael, Yorkshire LS11 8D (GB); KECMAN, Maja, Greater London E2 7LJ (GB); STROUX, Lisa, 69120 Heidelberg (DE); PELL, Randy, Marlboro VT 05344 (US); BENTLEY, Dan, Rochester NY 14623 (US)
(74) Representative: Alton, Andrew
(86) International application number: PCT/US2009/060205
(87) International publication number: WO 2010/042847

(56) References cited:
- EP-A2- 0 745 536
- WO-A1-2006/055083
- WO-A2-2009/018948
- DE-A1- 4 238 535
- US-A- 4 342 392
- US-A- 4 770 339
- US-A- 6 132 349
- US-B1- 6 594 971
- US-B1- 7 021 485
- US-B2- 6 793 879

## Description

### BACKGROUND OF THE INVENTION

### Field of the Invention

The present invention relates to a cover, and in particular to a cover for sterilizable medical containers to improve the handling, storage and organisation of such containers after sterilization.

### Description of Related Art

Reusable medical devices, such as instruments, need to be sterilized before re-use. Often steam based sterilization systems are used to sterilize the instruments. Sterilization systems typically include a container, such as a rigid exterior perforated case of either a metal or plastic material and having a rigid lid. Instruments are often held in a tray which is placed in the case. Multiple trays can be stacked within the same case. Common sterilization practices require the cases to be wrapped in a sheet or sheets of a wrapping material which provide a sterile barrier to maintain the sterility of the instruments after sterilization. The wrapping material, sometime referred to as "bio-wrap", is often provided as sheets of paper of fabric and one or more sheets of the wrap are used to wrap the cases prior to sterilization so that the outer wrapping maintains the sterility of the cases post sterilization.. This can create a large amount of disposable waste.

The sterilized wrapped cases are then placed in hospital storage rooms until transport to the operating room is required. However, the wraps are susceptible to rips and tears which compromise the sterility of the instruments within the case. Also, the uneven wraps tend to make it difficult to stack and organise the cases on the shelves in the storage rooms. Sometimes, when trying to remove a case from a stack of cases, the wrap around the case being removed or other cases in the stack can be damaged thereby compromising sterility. Also, other cases in the stack or in adjoining stacks can be dislodged from the shelves and fall to the floor again damaging the wrap and sometimes spilling the content of the cases. Handles placed on the exterior of cases are not accessible when the tray is wrapped. The wrapping material makes it difficult to transport wrapped containers and the lack of proper handles leads to ripping of the wrap. In an attempt to clasp onto the handle mechanism of the case under the wrap, fingers can penetrate the wrap and again compromise the sterility of the instrument case. Whenever the sterility has been compromised or possibly compromised, it is necessary to re-sterilize the case and contents which is a drain on the sterilization resources available to the hospital and can lead to understocking of sterilized instruments available for use.

Attempts at ameliorating these problems have focussed on removing the necessity for the use of bio-wraps through the use of a sterile container or a disposable filter bag in place of the wrapping material. However, many sterilization facilities still use the practice of wraps to maintain sterility during storage and transportation.

Therefore there is a need to improve the maintenance of sterility during storage and handling of wrapped sterile medical containers.

### SUMMARY OF THE INVENTION

A first aspect of the invention provides a cover for a sterilizable medical container wrapped in at least one sheet of a wrapping material, the cover comprising: a body including an upper part configured to hold the wrapping material against an upper portion of the container and a lower part configured to hold the wrapping material against a lower portion of the container, wherein the body can be assembled about the wrapped container to hold the wrapping material about the container; and at least one handle extending from the body which can be held by a user to move the cover when assembled about the wrapped container, characterized in that the cover comprises two separate members which are the same as each other and which can be joined together to assemble the cover about the wrapped container.

The cover goes around the outside of the wrapped container which acts to hold the wrap against the container and helps protect the wrap from damage and also includes a handle to facilitate handling of the wrapped container. Hence, the cover helps to reduce damage to the wrap material during storage and handling of the sterile containers and thereby helping to maintain their sterility and reduce the number of containers that need to be re-sterilized.

By at least partially covering the wrapping material, damage to the wrapping material can be reduced thereby helping to maintain the sterility of the container and its contents after sterilization.

By holding the wrapping material against the container, the cover also to keep the wrapped container more tidy, which helps to improve the handling, storage and manipulation of the covered container, thereby also helping to reduce damage to the wrapping material and hence improving sterility.

Further, by providing a handle as part of the cover, a user can use the handle to manipulate and mover the covered wrapped container and so the user does not need to handle the wrapping material itself, which yet further helps to reduce damage to the wrapping material and hence helps to maintain sterility.

As the two separate members are the same as each the other, only a single type of member needs to be manufactured and the members can be used interchangeably in a modular fashion.

The cover can be made of a sterilizable material. Hence, the cover can be applied before sterilization. Such as cover is also re-usable as it can be sterilized again after use.

The handle can be provided as an integral part of the body. This can simplify manufacture of the cover as the handle is formed as part of the main body of the cover.

The body can have a generally rectangular shape and the handle can be provided on the shorter side of the body or on the longer side of the body.

The cover can include a plurality of handles. Preferably the cover can includes two or at least two handles. A first handle can be provided at a first end of the body. A second handle can be provided at a second end of the body. The second end can be at an end opposite to the first end. A handle can be provided at each end of the body and at the ends separated by the longest dimension of the body.

The body can be permeable to steam. The material of the body can be made of a material which is itself permeable to steam. The body can include a plurality of apertures to make the body permeable to steam. This facilitates the ingress and egress of steam during sterilization when the cover is made of a material which is not itself permeable to steam.

The cover can include a fastener for securing the cover about the wrapped container. The fastener can secure the upper member and lower member together when the cover is assembled about the wrapped container. The fastener can be a single use fastener or the fastener can be a releasable fastener. The fastener can be a mechanical fastener or can be an adhesive fastener.

The cover can be made from a plastic material.

Instrument cases are often marked with information concerning the interior contents. After the case is wrapped this information is hidden. Hospital staff sometimes create labels to mark the interior contents of the wrapped container, however this manual process is tedious and prone to error. Therefore, the cover can bear one or more indicia indicating information relating to the contents of the container. The information can relate to a manufacturer of the contents, a model name of the contents, the type of the contents, the size of the contents, other attributes of the contents and any combination thereof.

Each member can be generally tray shaped. Each member can be generally rectangular with a low wall extending generally around its periphery. Each member can be made of a thermoformed plastic.

An outer surface of the cover can present a guide formation which allows another cover to be slid along a longitudinal axis of the cover.

An outer surface of the cover can present a guide formation which allows the self aligned stacking of covered wrapped containers.

An outer surface of the cover can present a guide formation which prevents sliding of another cover in a direction generally perpendicular to a direction along which the covers can be slid.

The guide formation can be in the form of at least one ridge and at least one groove. Preferably a plurality of ribs and grooves are provided. The ribs and grooves can be provided as an integral part of the body of the cover.

The cover can be generally dimensioned to receive a sterilizable medical container when at least partially covered by a wrapping material. The cover can be dimensioned to receive a container having dimensions in metres of height, width and depth selected from: 0.05 by 0.25 by 0.50; 0.10 by 0.25 by 0.50; 0.15 by 0.25 by 0.50; 0.05 by 0.25 by 0.25; and 0.10 by 0.25 by 0.25.

A further aspect of the invention provides a method for covering a sterilizable medical container wrapped in at least one sheet of wrapping material comprising the steps of: placing the sterilizable container wrapped in the wrapping material onto a first part of a cover; assembling a second part of the cover separate to the first part about the wrapped container so as to hold the wrapping material against the container, wherein the first part and the second part are the same as each other; and securing the cover about the wrapped container.

The method can further comprise sterilizing the wrapped container after being covered by the cover.

The wrapped container can already be sterilized before being covered by the cover.

### BRIEF DESCRIPTION OF DRAWINGS

An embodiment of the invention will now be described, by way of example only, and with reference to the accompanying drawings, in which:
Figure 1 shows a plan view of a first cover in the form of a cardboard foldable flat;
Figures 2A to 2D illustrate assembly of the cover shown in Figure 1 about a wrapped container;
Figure 3 shows a perspective view of a second cover;
Figure 4 shows a perspective view of a third cover;
Figure 5 shows a plan view of a fourth cover in the form of a plastics foldable flat;
Figure 6 shows a plan view of a fifth cover in the form of a plastics foldable flat;
Figure 7 shows a magnified view of a corner feature of the cover shown in Figure 6, when assembled in use;
Figure 8 shows a plan view of a sixth cover in the form of a plastics foldable flat;
Figure 9 shows a magnified view of a corner feature of the cover shown in Figure 8, when assembled in use;
Figure 10 shows a perspective view of an embodiment of a cover according to the invention when assembled in use;
Figure 11 shows a magnified view of a fastener feature of the cover shown in Figure 10; and
Figure 12 shows a plan view of a component part of the cover shown in Figures 10 and 11.

Similar items in different Figures share common reference numerals unless indicated otherwise.

### DETAILED DESCRIPTION OF THE INVENTION

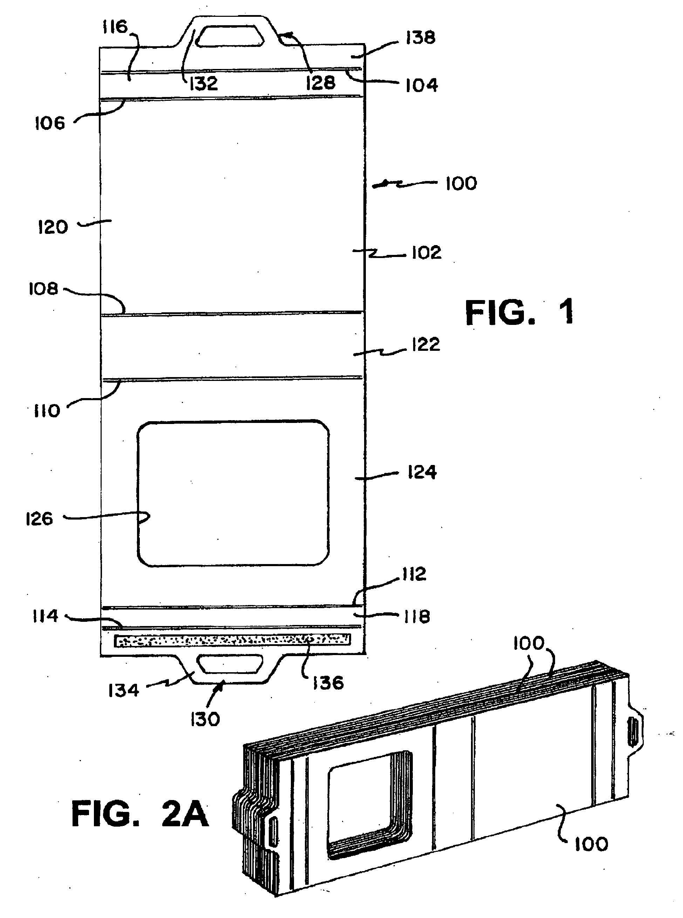

With reference to Figure 1 there is shown a first cover 100 for assembling about **a** wrapped sterilizable container. The container may be a case or similar, or simply a tray holding whatever is to be sterilized. In the following, the container will generally be used to refer to any structure which is used to hold or support whatever is being sterilized during the sterilization process. For example, the container may refer to a case with a one or more trays located therein or simply to a sterilizable tray, or similar, or multiple trays or similar.

The cover 100 is in the form of a foldable flat 102 made of a cardboard material. In other versions, the foldable flat may be made from a plastics material. The foldable flat 102 includes a number of fold formations 104, being parts of the cardboard flat which have been engineered are otherwise adapted to cause the cardboard material to preferentially fold along that line, in a desired direction. The engineered folds 104 to 114 are positioned on the flat so as to define portions corresponding to first and second side walls, and upper and lower portions or surfaces of the container to be covered. For example, portions 116 and 118 are dimensioned to provide a cover for a first side wall, portion 122 a cover for a second side wall, portion 120 a cover for a lower portion of the container and portion 124 a cover for an upper portion of the container. Portion 124 also includes an aperture 126 therein which provides a window via which information about the contents of the container on a printed sheet can be viewed.

A first handle portion 128 and a second handle portion 130 are provided at first and second ends of the flat respectively. Each handle portion includes a handle formation 132, 134 which co-operate and assembled to provide a handle for the cover. Handle portion 130 also includes a strip of adhesive material 136. The adhesive material can be used to provide a fastener to secure the cardboard flat in its assembled configuration when assembled about a wrap container in use. Adhesive material 136 can be a contact adhesive which adheres to a corresponding part 138 of handle portion 128. In alternative versions, adhesive 136 may be covered by a releasable cover strip which is removed prior to assembly of the cover 100.

As described above, it will be appreciated that the cover generally has a main body section with an integral handle formations 132 and 134. The foldable flap 102 can easily be manufactured simply by a stamping process in which the apertures are created by a cutting instrument acting on a sheet of cardboard material and in which the fold lines 104 to 114 are created by a scoring, or similar, process. In an alternate version, portions of the body of the foldable flap 102 are provided with a plurality of apertures arranged generally all over the body in order to allow the ingress and egress of steam during sterilization.

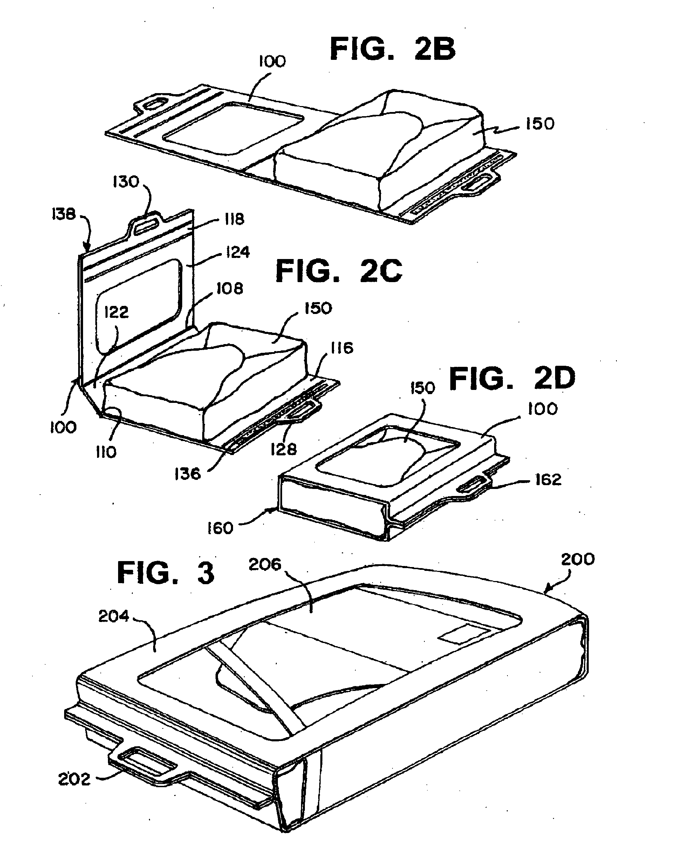

With reference to Figures 2A to 2D, use of the cover 100 will be described in greater detail. Figure 2A shows a plurality of covers 100 as illustrated in Figure 1. A cover 100 in an unassembled state is selected and the sterilized container covered by at least one sheet of wrapping material 150 is placed on the lower part 120 of the cover 100, as illustrated in Figure 2B. As illustrated in Figure 2C, assembly of the cover is commenced by folding the cover along fold line 108 and 110 so that portion 122 of the cover engages a rear side of the wrapped container and eventually, by further folding cover along line 110, the upper portion 124 of the cover is brought into contact with the upper portion of the container. Portions 116 and 118 of the cover are folded down to engage a forward side of the wrapped container and handle portions 128 and 130 are folded and engaged together and adhesive 136 is brought into engagement with region 138 and thereby secures the cover in its assembled formation about the wrapped container as illustrated in Figure 2D. Hence, the end result is an assembly 160 comprising the cover 100 assembled about the wrapped container 150 as illustrated in Figure 2D.

As can be seen, the cover holds the sheet of wrapping material about the container and also at least partially protects the wrapping material by generally enclosing it. The cover 100 also presents a handle 162 by which the container can be more easily handled during storage or movement by a user.

As illustrated in Figure 1, the first cover 100 has a generally rectangular shape and the handle extends along a one of the longer sides of the cover. Figure 3 shows a second version of the cover 200, generally similar to the first version shown in Figure 1. Cover 200 also has a generally rectangular shape, but handle 202 extends along and from a shorter side of the cover 200. As also illustrated in Figure 3, aperture 204 in the upper part of cover 200 provides a window by which a user can view information about the contents of the sterilized container presented on a sheet of paper 206.

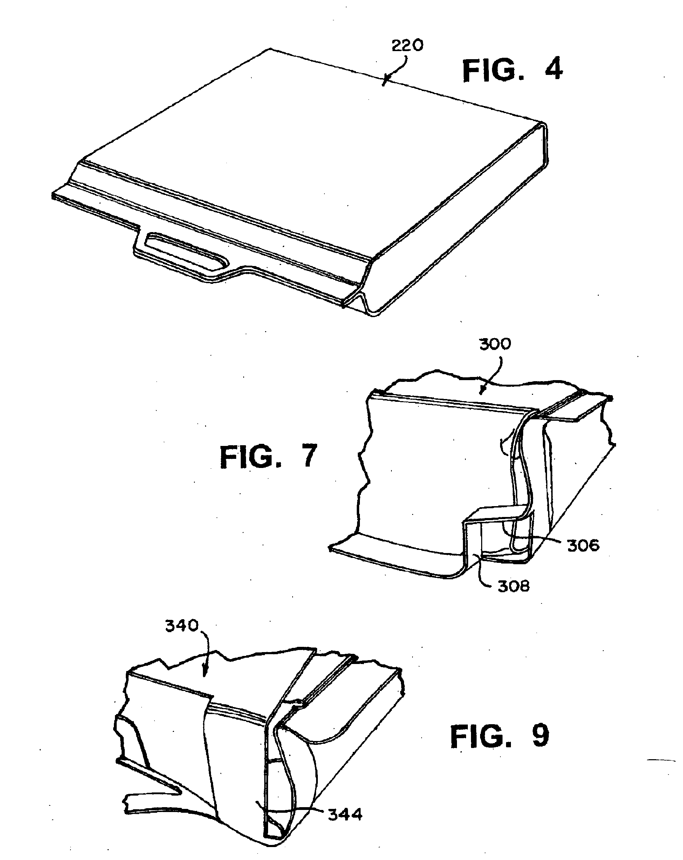

Figure 4 illustrates a third version 220 of the cover. This version is similar to the first version except that it does not include a window aperture. As also illustrated in Figure 4, the cover can bear at least one indicium on an outer surface of the cover 220 which provides information about the content of the container. For example, the cover 220 can include an indication of the manufacturer of the contents, the type or nature of the contents, for example, the name or type of an instrument, information about the size of the contents and/or any other properties or attributes of the medial instruments or devices held in the container.

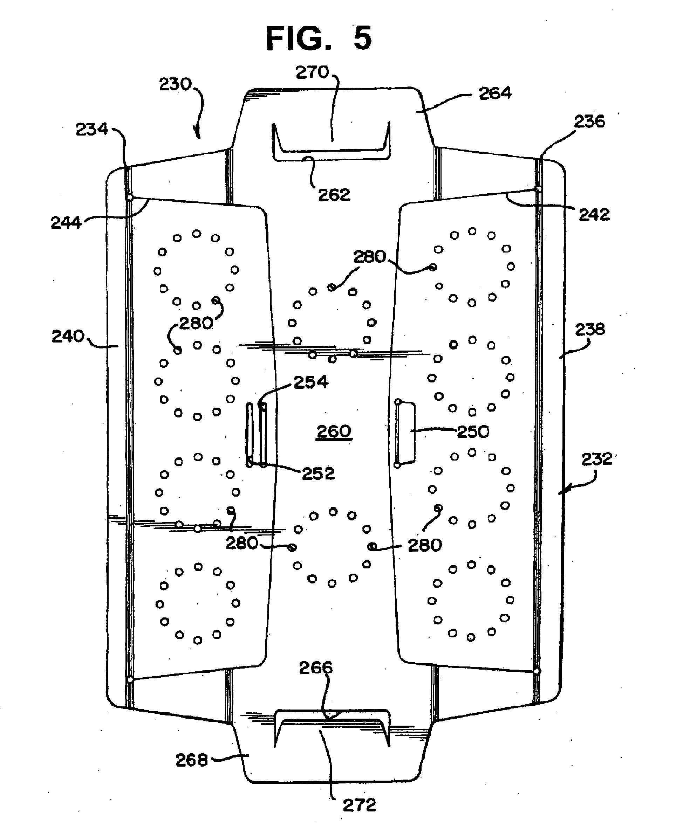

With reference to Figure 5, there is shown a plan view of a fourth version 230 of a cover. The cover 230 is in the form of a foldable flat 232 made of a plastics material, such as polypropylene, or a thermoformable material or plastic The cover 230 is shown in an unassembled state and is assembled about a wrap container in use, as will be described in greater detail below.

The plastics flat 232 includes a number of apertures and cuts in the plastics material and also includes engineered fold formations, e.g. 234, 236 to allow the flat to be folded along preferred directions. A first lip 238 extends along a first side of the flat and a second lip 240 extends along a second posed side of the flat. A first cut line 242 extends from fold formation 236 at one end of the flat to the other opposed end of the flat and defines a first half of an upper portion of the cover. A second similar cut 244 extends from fold formation 234 generally and defines a second half of the upper portion of the cover 248. The first half 246 includes a cut defining a flap or tongue member 250. Second half 248 includes a pair of slots 252, 254, positioned to receive flap 250 when the cover is assembled about a wrap container in use. Between them, flap 250 and slots 252, 254 provide a fastener for folding the cover about a wrapped container.

A central portion 260 of the flat 232 provides a lower portion of the cover for engaging a lower part of the wrapped container in use.

A first generally U-shaped aperture 262 is provided in a handle portion 264 at a first end of the flat. A second generally U-shaped aperture 266 is provided in a second handle portion 268 at a second end of the flat. U-shaped apertures 262, 266 define respective flaps 270, 272 which can be folded out of the plane of the flat 232 to provide hand holds for a user to grip a handle provided by the end handle portions 264, 268 of the flat.

The flat includes a plurality of apertures e.g. 280, arranged in ten circular patterns about the flat so as to allow the ingress and egress of steam through the material of the flat during sterilization.

In use, the first and second halves 246, 248 of the upper portion of the cover are folded out of the plane of a flat along fold formations 234 and 236 and the wrap container is placed on the lower part 260 of the cover between the first and second halves 246, 248. The first and second halves are then drawn toward each other which causes the flat to become assembled and wrap around the wrapped container and flap 250 is inserted into slot 252 or 254 in order to fasten the first and second halves 246, 248 of the flat to form the upper portion of the cover on the upper part of the wrapped container.

Hence, in this assembled state, the cover generally wraps around the wrapped container so as to hold the wrap against the container and also generally protect the wrap from damage.

In order to facilitate handling of the covered wrapped container, a user folds flaps 272, 270 to insert their hands into hand holds in the handle portions and can then transport the covered wrapped container to a sterilization facility for sterilization. The presence of the plurality of apertures, e.g. 280, allows the easy passage of steam into and out of the container via the wrap material. After sterilization, the handles can again be used by a user to facilitate handling of the sterilized container and contents and the sterilized container and contents within the wrap and cover can be transported to and stored in a storage facility until required in future. As will be appreciated, the cover helps to manage the fabric wrap and also provides substantially flat surfaces to facilitate stacking of multiple covered wrap containers in an easy manner. Also, the provision of handles allows a user to easily select the covered wrapped container from a stack of such containers and handle the container without causing damage to the wraps of other similar containers.

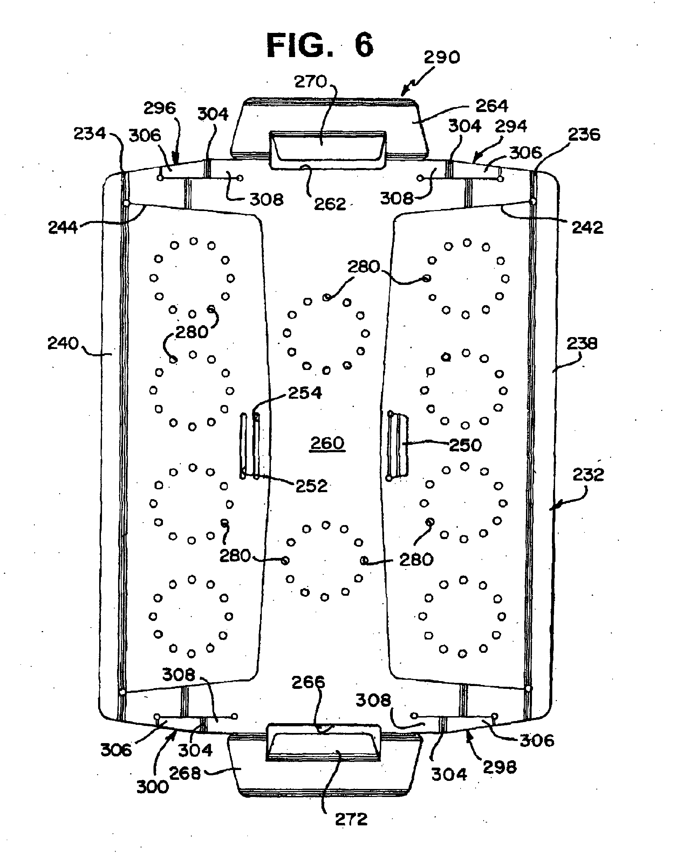

With reference to Figure 6, there is shown a fifth version of a cover 290. The fifth version 290 is generally similar to the fourth version 230 and is also provided in a form of a foldable plastics flat 292. Figure 6 shows further fold formations not necessarily shown in Figure 5. The flat also includes a mechanism to retain the wrapped within the foldable flat to prevent the container sliding out of the cover along the longitudinal axis of the cover. The mechanism is illustrated in greater detail in Figure 7 which shows a perspective view of the cover 290 when assembled in use about a wrapped container 300. A illustrated best in Figure 6, towards each of the four corners or shoulders of the flat 294, 296, 298, 300, a retaining formation is provided by a cut and a fold formation, e.g. cut 302 and fold formation 304. When the cover is assembled about the wrapped container, generally as described above, the retaining formation is provided by folding a strip of plastics material about fold formation 304 so as to provide a barrier extending from the lower part of the cover to the side wall of the cover so as to prevent any movement of the wrap container along the longitudinal axis of the cover.

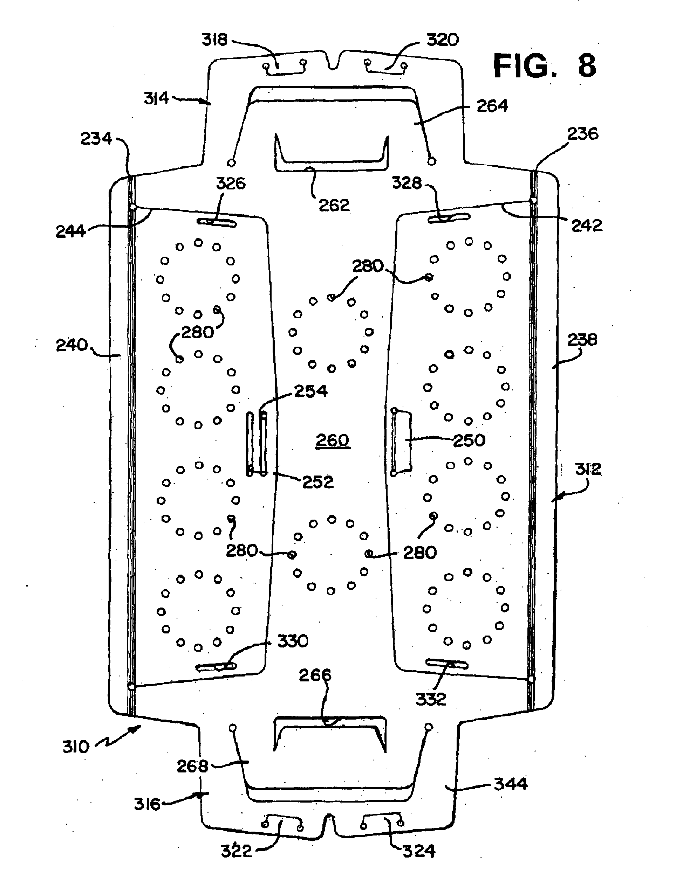

Figure 8 shows a plan view of a sixth version of the cover 310 generally similar to the versions shown in Figures 5 and 6. Again, the cover 310 is provided in the form of a foldable plastics flat 312 and is shown in Figure 8 in an unassembled configuration. Cover 310 is similar to cover 290 in that it includes a retention mechanism to help prevent longitudinal sliding of the wrapped container within the cover in use. A retention mechanism is provided at each end of the cover. The retention mechanism takes the form of a generally U-shaped portion 314, 316 of the flat which can be folded up from the central portion 260 of the flat and which each bears a pair of tongues 318, 320, 322, 324. A slot 326, 328, 330, 322 is provided toward each end of each half of the upper portion of the cover for receiving a one of the tongues in use.

Figure 9 shows a magnified perspective view of a corner of the cover 310 when assembled in use about a wrapped container 340. After the first and second halves of the upper portion have been drawn together and fastened by tongue 250 in one of slots 252, 254, a retaining mechanism is provided by folding the U-shaped members 314, 316 upward out of the plane of the flat and inserting tongues 318 and 320 in slots 326 and 328 respectively and tongues 322, 324 in slots 330 and 332 respectively. By doing so, end retaining members, e.g. portion 344 provides a strap which engages about the end of the wrapped container to restrict its movement along the longitudinal axis of the cover.

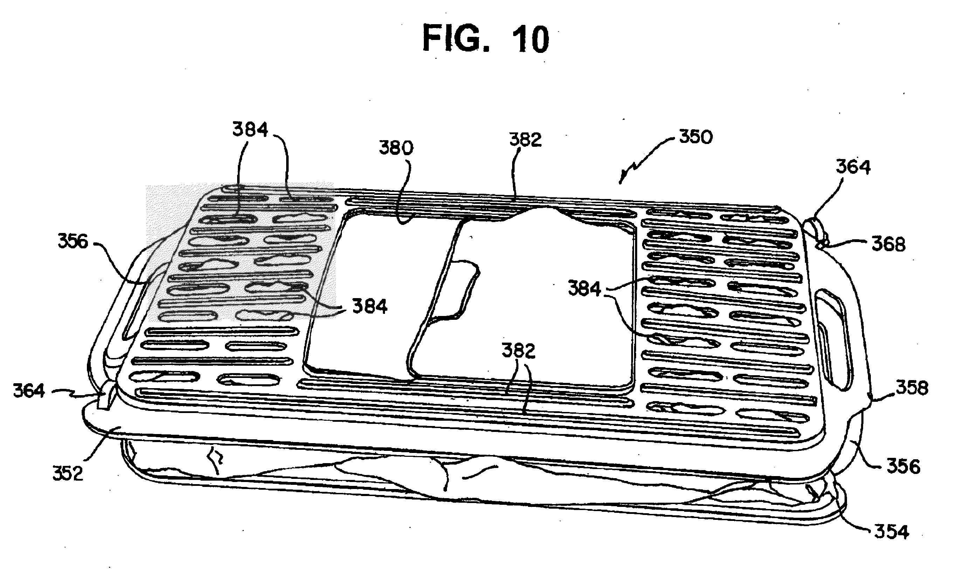

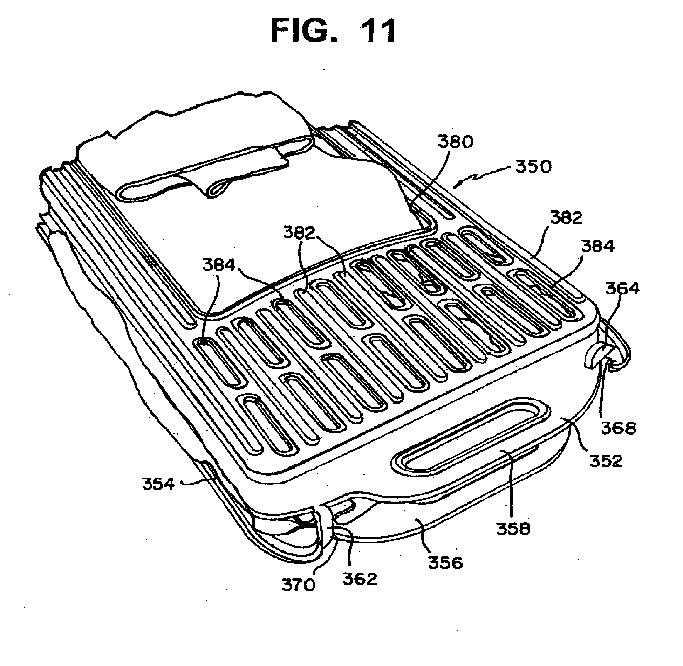

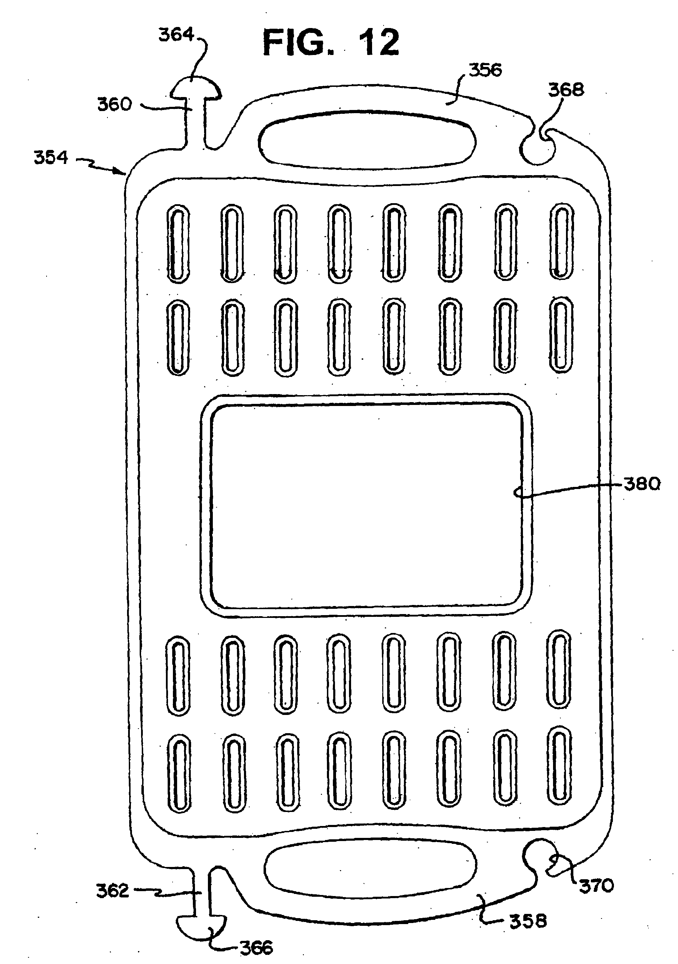

With reference to Figures 10 and 11 there are shown perspective views of an embodiment of a cover 350 according to the invention. The cover 350 is in the form of a pair of generally tray-shaped members 352, 354, a one of which is schematically illustrated in plan view in Figure 12. Each generally tray-shaped member 352, 354 has a main generally flat section with a short wall extending up from and around the periphery thereof. A handle portion 356, 358 extends from each end of the main body of the cover. At each end, to a first side of the handle portion, a first part of a fastening mechanism is provided in the form of a strap 360, 362, terminating in a head portion 364, 366. To the opposite side of each handle portion, a generally C-shaped or re-entrant aperture 368, 370 is provided with a narrow entrance. The aperture provides a second part of the fastener mechanism and is configured to receive and retain therein the strap portion of the fastening mechanism when assembled about a wrapped container in use.

The main body of the cover includes a central large rectangular aperture 380 which provides a window via which information can be viewed. The main body of the cover also includes a plurality of lozenge or oval shaped apertures to allow the ingress and egress of steam during sterilization. As best illustrated in Figures 10 and 11, the sequence of convex ribs and concave grooves are provided on the outer surface of each tray member. The rib and grooves alternate and are dimensioned so that a rib from a one cover will mate with a groove from another cover on which it is stacked so as to allow the stacked covered containers to be slid laterally relative to each other but to prevent sliding in a direction generally transverse to the longitudinal axis of the cover, as will be described in greater detail below.

The cover is made up from two identical tray portions, as illustrated in Figure 12, with a first of the tray portions receiving the wrapped container therein and a second tray portion inverted and placed over the top part of the wrap container as illustrated in Figures 10 and 11. Between them the tray parts provide the main body of the wrapper with the handle formations providing a handle at each end of the cover which is integral to the main body of the cover. The tray members are identical and so can be used inter-changeably in a modular fashion. Hence, it is only necessary to manufacture one type of member which can then be used as either the upper or lower part of the cover. The parts of the cover are fastened together in the assembled state by engaging stems 360, 362 in corresponding apertures in the upper inverted tray and engaging similar stems in the upper inverted tray into the apertures 368, 370 in the lower tray.

Use of cover 350 is generally similar to use of the other covers as described above. The wrapped container is placed in a lower tray and an inverted upper tray is placed over the wrapped container and the parts fastened together to assemble the cover about the wrap container. That assembly can then be sterilized with the plurality of apertures in the main body of the cover allowing the easy passage of steam into and out of the wrapped container. After sterilization, the assembly can be moved using the handles and the sterilized assembly stored in stacks of similar assemblies in a storage facility. As mentioned above, the outer surfaces of the upper and lower trays present a number of convex ribs, e.g. 382, and a number of concave similarly shaped troughs, e.g. 384. Similarly shaped ribs and troughs are provided between the plurality of apertures either side of the main viewing aperture 380. When stacked, the ribs and troughs of the upper surface of a lower tray can engage with the troughs and ribs respectively of a lower surface of an adjacent upper tray. The ribs and troughs extend generally along the longitudinal axis of the cover and are generally elongate and straight and facilitate the sliding of trays relative to each other in the longitudinal direction. This can facilitate the removal of a tray from within a stack of trays without interrupting the stability of the stack.

Also, the interaction of the ribs and troughs help to prevent sliding of trays within a stack of trays in a direction generally transverse to the longitudinal axis of the cover. For example, if a stack of several assemblies is being carried to or from the store room, then this helps to prevent trays from sliding off the stack in a transverse direction which can otherwise happen.

The interaction between the ribs and troughs, or ridges and grooves, also helps to provide self-alignment of the stacked trays. Further, the grooves provide an air space which improves the effectiveness of the sterilization. As discussed above, the interaction of the ridges and grooves also provides stability for stacks of trays during transportation.

This cover is particularly preferred as it is quicker and easier to assemble about a wrapped container than the other covers. Also, it is easily adjustable to accept wrapped containers of different sizes. As would be appreciated, as each tray component is identical, it is only necessary to fabricate a single type of item and then each tray can be used with similarly shaped trays in a modular system in order to provide the cover.

In practice, the tray can be thermoformed from a thermoplastic material, such as polypropylene, and the tray can be thermoformed using a heat based moulding process. A preferred gauge for the polypropylene material is approximately 0.04 to 0.06 inches. Other suitable thermoformable plastics which can be used to fabricate the cover include polyethylene and acrylonitrile butadiene styrene (ABS).

As will be appreciated from the above discussion of the invention, it provides a number of advantages and solves a number of problems associated with wrapped sterilizable medical containers. The cover of the invention helps to retain the wrapping material about the container in a tidy manner which helps to facilitate handling and storage of the wrapped containers. Also, the cover serves to at least partially protect the wrapping material thereby helping to reduce the incidence of damage or tearing of the wrapping material. Further, the covers provide a generally flat surface which further facilitates the storage by stacking of multiple wrapped containers. Further, the covers facilitate access to information about the contents of the wrapped container so that it is easier to ensure that the correct sterilized contents are retrieved from a store when required. Yet further, the integral handle further facilitates the handling and manipulation of the wrap container so there is no need to grasp the wrapping material or try to access any handle on the container as the cover provides an easily accessible handle to a user.

The exact dimensions of the cover will depend on the size of container or tray with which it is intended to use the cover. For example, sterilizable containers or trays are generally provided with dimensions of height by width by depth or approximately 5 x 25 x 50, 10 x 25 x 50, 15 x 25 x 50, 5 x 25 x 25 and 10 x 25 x 25 (all measurements in centimetres). It will be appreciated that the cover can be used with other sizes of sterilization trays or containers as required. Therefore, the dimensions of the cover will generally match the dimensions of the container to be covered, but with some tolerance to accept the sheet or sheets of wrapping material in which the container has been wrapped.

In other embodiments of the invention, the cover can be dimensioned to accept multiple containers therein. For example, a cover can be provided which can accept a stack of individual trays or containers. Additionally or alternatively, covers can be provided with fastening mechanisms which allow the size of the cover to be adjusted depending on the size of the container to be covered. For example, the fastening mechanism of the embodiment could include a longer stem portion 360, 362 with multiple sets of lugs 364, 366 extending from the sides thereof so as to allow the pair of members of the cover to be secured together at different vertical separations. Hence, different height stacks of containers could be received within a single cover.

## Claims

1. A cover (350) for a sterilizable medical container wrapped in at least one sheet of a wrapping material, the cover comprising:
a body including an upper part configured to hold the wrapping material against an upper portion of the container and a lower part configured to hold the wrapping material against a lower portion of the container, wherein the body can be assembled about the wrapped container to hold the wrapping material about the container; and
at least one handle extending from the body which can be held by a user to move the cover when assembled about the wrapped container, **characterized in that** the cover comprises two separate members (352, 354) which are the same as each other and which can be joined together to assemble the cover about the wrapped container.

2. The cover (350) of claim 1, wherein the handle is provided as an integral part of the body.

3. The cover (350) of claim 1, wherein the cover includes two handles, with one handle at a first end of the body and a second handle at a second end of the body, opposite to the first end.

4. The cover of claim 1, wherein the body includes a plurality of apertures to allow the ingress and egress of steam during sterilization.

5. The cover (350) as claimed in claim 1, wherein each member is tray-shaped.

6. The cover (350) as claimed in claim 3, wherein each member has a handle portion (356, 358) extending from each end of the member and which provide the two handles.

7. The cover as claimed in claim 5, wherein each member is thermoformed from a thermoplastic material.

8. The cover as claimed in claim 1, wherein each member includes a main viewing aperture (390) providing a window via which information can be viewed.

9. A method for covering a sterilizable medical container wrapped in at least one sheet of wrapping material comprising the steps of:
placing the sterilizable container wrapped in the wrapping material onto a first part of a cover;
assembling a second part of the cover separate to the first part about the wrapped container so as to hold the wrapping material against the container, wherein the first part and the second part are the same as each other; and
securing the cover about the wrapped container.

10. The method of claim 9 and further comprising sterilizing the wrapped container after being covered by the cover.

11. The method of claim 9, wherein the wrapped container is already sterilized before being covered by the cover.

## Patentansprüche

1. Abdeckung (350) für einen sterilisierbaren medizinischen Behälter, der von mindestens einer Lage aus einem Verpackungsmaterial umhüllt ist, wobei die Abdeckung umfasst:
einen Körper, der einen oberen Teil, der konfiguriert ist, um das Verpackungsmaterial gegen einen oberen Abschnitt des Behälters zu halten, und einen unteren Teil, der konfiguriert ist, um das Verpackungsmaterial gegen einen unteren Abschnitt des Behälters zu halten, enthält, wobei der Körper um den umhüllten Behälter montiert werden kann, um das Verpackungsmaterial um den Behälter zu halten; und
mindestens einen Griff, der sich von dem Körper erstreckt und von einem Benutzer gehalten werden kann, um die Abdeckung im um den umhüllten Behälter montierten Zustand zu bewegen, **dadurch gekennzeichnet, dass** die Abdeckung zwei separate Elemente (352, 354) aufweist, die zueinander gleich sind und miteinander verbunden werden können, um die Abdeckung um den umhüllten Behälter zu montieren.

2. Abdeckung (350) nach Anspruch 1, wobei der Griff als ein integraler Teil des Körpers vorgesehen ist.

3. Abdeckung (350) nach Anspruch 1, wobei die Abdeckung zwei Griffe enthält, wobei sich ein Griff an einem ersten Ende des Körpers und ein zweiter Griff an einem zweiten Ende des Körpers, gegenüber dem ersten Ende, befindet.

4. Abdeckung nach Anspruch 1, wobei der Körper eine Vielzahl von Öffnungen für den Eintritt und Austritt von Dampf während Sterilisation enthält.

5. Abdeckung (350) nach Anspruch 1, wobei jedes Element tablettförmig ist.

6. Abdeckung (350) nach Anspruch 3, wobei jedes Element einen Griffabschnitt (356, 358) aufweist, der sich von jedem Ende des Elements erstreckt und die die beiden Griffe bereitstellen.

7. Abdeckung nach Anspruch 5, wobei jedes Element aus einem thermoplastischen Material warmgeformt ist.

8. Abdeckung nach Anspruch 1, wobei jedes Element eine Hauptbeobachtungsöffnung (390) enthält, die ein Fenster bereitstellt, über das Information betrachtet werden kann.

9. Verfahren zur Abdeckung eines sterilisierbaren medizinischen Behälters, der von mindestens einer Lage von Verpackungsmaterial umhüllt ist, umfassend die Schritte:
Platzieren des sterilisierbaren Behälters, der in das Verpackungsmaterial eingehüllt ist, auf einem ersten Teil einer Abdeckung;
Montieren eines zweiten Teils der Abdeckung separat zum ersten Teil um den umhüllten Behälter, um das Verpackungsmaterial gegen den Behälter zu halten, wobei der erste Teil und der zweite Teil miteinander gleich sind; und
Sichern der Abdeckung um den verpackten Behälter.

10. Verfahren nach Anspruch 9, ferner umfassend Sterilisieren des verpackten Behälters, nachdem er von der Abdeckung bedeckt ist.

11. Verfahren nach Anspruch 9, wobei der umhüllte Behälter bereits sterilisiert ist, bevor er von der Abdeckung bedeckt wird.

## Revendications

1. Couvercle (350) pour un récipient médical pouvant être stérilisé, enveloppé dans au moins une feuille de matériau de conditionnement, le couvercle comprenant :
un corps incluant une partie supérieure configurée pour retenir le matériau de conditionnement contre une portion supérieure du récipient et une partie inférieure configurée pour retenir le matériau de conditionnement contre une portion inférieure du récipient, où le corps peut être assemblé autour du récipient enveloppé pour retenir le matériau de conditionnement autour du récipient ; et
au moins une poignée s'étendant du corps qui peut être tenue par un utilisateur pour déplacer le couvercle lorsqu'il est assemblé autour du récipient enveloppé, **caractérisé en ce que** le couvercle comprend deux éléments séparés (352, 354) qui sont identiques et qui peuvent être joints ensemble pour assembler le couvercle autour du récipient enveloppé.

2. Couvercle (350) selon la revendication 1, où la poignée est réalisée comme une partie intégrale du corps.

3. Couvercle (350) selon la revendication 1, où le couvercle comprend deux poignées, une poignée se trouvant à une première extrémité du corps, et une deuxième poignée à une seconde extrémité du corps, opposée à la première extrémité.

4. Couvercle selon la revendication 1, où le corps comprend une pluralité d'ouvertures pour permettre l'entrée et la sortie de la vapeur durant la stérilisation.

5. Couvercle (350) selon la revendication 1, où chaque élément est en forme de plateau.

6. Couvercle (350) selon la revendication 3, où chaque élément possède une portion de poignée (356, 358) s'étendant de chaque extrémité de l'élément et qui réalise les deux poignées.

7. Couvercle selon la revendication 5, où chaque élément est thermoformé à partir d'un matériau thermoplastique.

8. Couvercle selon la revendication 1, où chaque élément comprend une ouverture de vision principale (390) réalisant une fenêtre par laquelle des informations peuvent être vues.

9. Procédé pour couvrir un récipient médical pouvant être stérilisé enveloppé dans au moins une feuille de matériau de conditionnement, comprenant les étapes de :
placer le récipient apte à être stérilisé enveloppé dans le matériau de conditionnement sur une première partie d'un couvercle ;
assembler une deuxième partie du couvercle séparée de la première partie autour du récipient enveloppé de manière à tenir le matériau de conditionnement contre le récipient, où la première partie et la deuxième partie sont identiques ; et
fixer le couvercle autour du récipient enroulé.

10. Procédé selon la revendication 9 et comprenant en outre la stérilisation du récipient enveloppé après qu'il a été couvert par le couvercle.

11. Procédé selon la revendication 9, dans lequel le récipient enveloppé est déjà stérilisé avant d'être couvert par le couvercle.
